# EUROPEAN PATENT APPLICATION

(11) **EP 3 494 980 A1**
(43) Date of publication of application: **12.06.2019**
(21) Application number: 18209780.8
(22) Date of filing: 03.12.2018
(51) Int. Cl.: A61K 35/646, A61P 35/00

(54) **SPIDER WEB EXTRACT, METHOD OF PREPARATION, AND USE FOR TREATING PROSTATE CANCER**

(30) Priority: 08.12.2017 TR 201719916
(71) Applicant: Doganlar, Oguzhan, Edirne (TR); Ozkan, Asli Nur, Edirne (TR)
(72) Inventor: Doganlar, Oguzhan, Edirne (TR); Ozkan, Asli Nur, Edirne (TR)
(74) Representative: Inal, Aysegul Seda

(57) **Abstract**

The invention is related to a method for obtaining spider web extract by grinding the spider web collected from nature by using liquid nitrogen and balls, precipitating the spider web powder in a solvent containing ethyl acetate, hydrochloric acid and dimethyl sulphoxide and thus obtaining a mixture containing a liquid extract fraction and a solid fraction; and then optionally mixing the solid or the semi-solid extract with a polar rearrangement solvent containing dimethyl sulphoxide, water, n-butanol, isopropanol, n-propanol, ethanol and methanol or a mixture thereof and said obtained spider web extract has the selective and targeted anti-cancer effect by increasing the production of reactive oxygen species and creating DNA damage and protein damage (Figure 4) in prostate cancer cells (Figures 2 and 3) without creating a lethal effect on healthy cells (Figure 1), then leading to a programmed cell death through intrinsic and extrinsic apoptosis pathways (Figures 8 and 9) by activating cell control pathways (Figure 7).

## Description

### Technical Field

This invention is related to extraction methods of spider web which is one of the strongest and most impressive engineering products of the nature without harming the living organism and also related to creating a strong oxidative stress on the prostate cancer cells, increasing DNA damage, inhibiting NF-kB gene and protein expression, stimulating cell cycle control mechanisms and creating a strong anti-cancer effect by triggering intrinsic and extrinsic programmed cell death mechanisms by the derived spider web extract.

### Background of the Invention

Today, even though a large sum of money is spent on studies and hundreds of researches are performed each year, cancer is still the 2^{nd} most important cause of death after ischemic heart diseases. According to the 2012 reports of World Health Organization, each year 14 million people are diagnosed with cancer and also according to the 2015 reports; each year 8.8 million people die due to cancer. Globally, 1 of each 6 death is related to cancer. Chemotherapy treatment which has started with general cytotoxic agents in 1940s and which has continued with potential chemicals of natural origin such as anthracycline and alkaloid obtained from vinca in 1960s is still the most effective method of fighting cancer. Now, in the modern fight with cancer, approximately 50 different types (under tens of different chemical names and structures) of chemotherapeutic agents are used on 200 different types of cancer.

Cancer cells are the cells that can continuously and uncontrollably reproduce, that subject the neighboring cells to assimilation through created signals and micro environmental conditions, that can spread quickly by occupying other tissues and organs and that form a strong resistance against cell death. These properties bring along big problems against developing successful anticancer treatments. The ability of cancer cells for escaping from cell death mechanisms, which they can develop quickly and in a very short time when compared to normal cells, makes it necessary to develop treatments focused on targeting the vulnerable aspects of the cancer cells to induce programmed cell death (Apoptosis) in cancer cells by minimally harming the healthy cells.

Programmed cell death is known as Apoptosis. Apoptosis is way of cell death required for regular cell development and homeostasis and is a defense mechanism of the body for disposal of damaged cells. Apoptosis is a process that has been learned in the evolutionary process for preventing the irreparable DNA, organelle and/or protein structure defects from transferring to new generation of cells, wherein the cell kills itself without harming the body by activating a set of gene and protein products and wherein these dead structures are digested in a controlled manner. Apoptosis is cell-specific and other cells are not affected from this death. In its basic form, apoptosis is divided into two types, that is intrinsic (mitochondrial) and extrinsic (death receptors) apoptosis pathways.

Mitochondrial apoptosis pathway is also known as the intrinsic pathway. In this pathway that creates a programmed cell death in cancer cells, the most important protein is the BCL gene family which is responsible for the permeability of the mitochondrial membrane structure. BCL is coded by a big gene and the header of it is responsible for coding the BCL-2 protein. This protein functions as an apoptosis inhibitor. This protein is closely related to tumor suppressor P53 gene that is over expressed when there is a non-lethal but irreparable DNA damage in the cancer cells and when the cells start reproducing uncontrollably. The quick increasing of P53 expression in need of apoptosis activates the PUMA and NOXA genes in the mitochondrial pathway and these genes suppress the activity of apoptosis inhibitor BCL-2 gene. After this suppression, the expression of BAX gene located at the end of the BCL gene starts increasing. BAX is a proapoptotic protein and increasing its density disrupts the mitochondrial membrane potential and permeability. This situation triggers the apoptosis mechanism and the Cytochrome-C in the mitochondria start flowing into the cell cytosol due to disrupted membrane potential. The Cytochrome-C quickly increasing in the cell cytosol joins with the APAF-1 protein which is present in the cell cytosol and of which the expression is increased in apoptosis. Later, Caspase 9 joins this protein structure and forms apoptosis structures named apoptosome. At this phase, apoptosomes trigger the Caspase 3 protein and the cell starts dying as a result of the increase in Caspase 3 expression. Thus, the mitochondrial apoptosis cycle is completed. Extrinsic apoptosis pathway is also known as death receptor signalization. Death can happen due to a plurality of pathways, however one of the well-known ones is the programmed cell death caused by activation of death receptor 4 and 5 (DR4-DR5) through increasing of TRAIL gene expression, triggering of Caspase 8 expression by these activities and then the quick increase of Caspase 3 expression. Cancer cells have developed strong mechanisms that prevent said apoptosis process for sustained reproduction and for occupying other cells. The cancer cells prevent especially the activation of P53 gene, prevent disruption of membrane potential of the mitochondria by regulating BCL-2 gene expression, cause these damages in the cancer cells to be overlooked by the control genes by suppressing the cell cycle control genes such as P21 and P27 and also stop beginning of the apoptosis through Nuclear factor Kappa B (NF-kB) activity.

NF-kB transcription factors family comprises important regulatory proteins which affect almost every property of the cell adaptation including the reactions against stress, inflammatory reactions, activation of immunity cell function, cellular reproduction, programmed cell death (apoptosis), differentiation and oncogenesis. NF-kB regulates more than 150 genes including cytokines, cell adhesion molecules and growth factors. There is a high NF-kB regulation in the cancer cells and thus the apoptosis process is controlled as desired by the cancer.

Nowadays, the chemotherapy agents used and developed in cancer therapies must defeat the cancer survival strategies in at least one of the above-mentioned mechanisms to kill cancer cells in the beginning, phase, in-effect phase or in the terminal phase. In the war with cancer, in order to overcome these strategies of the cancer, thousands of research are made through cooperative effort of many science disciplines from antineoplastic agents to nanomaterial-based medicine and from specific proteins to gene therapies and each year drugs with minimal toxic effect on healthy cells specific to targeted points in cancer are developed. Today, unfortunately, use of these drugs in chemotherapy of all cancer types is still less than 2%. In development of these drugs, as at is for thousands of years, the most important source is the natural products. The statistics of today show that 80% of the developed or developing population on earth depends 75% of the time on the natural products and also on the drugs that can be derived thereof which can be approved by FDA (Food and Drug Administration) for treatment of diseases including cancer and that currently there is a natural product market worth approximately of 300 billion dollars all over the world.

For centuries, in the Asian culture, the people have been fed with a diet rich in natural products for a long life. In the conducted researches, it was observed that one of the reasons why these people are healthy is that there is an abundant number of flavonoids present in the food they consume. Flavonoids that are generally present in the marine species, bacteria, weeds, seeds, vegetables and fruits are a vast nutritional family having anti-cancer, anti-viral, anti-oxidant properties. However, flavonoids are not the only material group in natural products that exhibits anti-cancer effect. There are hormones that exhibit anti-cancer properties by acting on the stress mechanism such as vegetative melatonin and Abscisic acid (ABA). It has been designated that many of these materials trigger the extrinsic apoptosis (Tnf-α-Caspase 8-Caspase 3) and intrinsic apoptosis (Mitochondrial pathway) pathways specifically in cancer cell series. Besides, it has been determined that materials such as Quercetin, Naringin, Gallic acid and Caffeic acid phenyl ester suppress both the apoptosis activator and the Nf-Kβ pathway. NF-kB pathway was shown as the target pathway for many drugs in cancer treatment of different cancer types where it is tried. While natural sources are shown to have anti-cancer properties on different cancer types on their own, current studies present that, when used in combination with chemotherapeutic agents on cancer cells, they exhibit properties such as ingesting drugs with cytotoxic effects into the cell, increasing the effects of the used agents and forming chemo sensitization in cells where multiple drug resistance has been developed and studies on this subject is continued. The natural products comprise several bioactive chemicals that can perform certain roles especially in cancer treatment. While their complex compositions and strong pharmacological properties trigger a specific target and impact mechanism in the cancer cells, these compounds which are already formed in the living organism without harming the living generally do not create a toxic effect on the healthy cells. Due to these properties, they are the essential and most promising components of the cancer research.

The spider web is one of the most perfect engineering wanders of the nature created by some of the spiders included in the Araneida team of the Arachnida class for purposes such as hunting, laying eggs, mating and building a nest. It is the strongest thread present in the nature and it is a natural product formed by special proteins that harden when come in contact with air and that are produced in liquid form from the three pairs of web organs which are located at the end of the bottom of the spiders' abdomens and which comprise tens of micro channels. In addition to proteins, several structural elements, polyphenols, hormonal compounds and vitamins are detected in its structure. Today, due to its strength and elasticity properties, spider webs are produced in the spider web farms established especially in America and Australia and they are used in the production of several special textile and clothing and particularly for production of steel vast which require intense strength. Besides, since the old ages, they are used for treatment of sword wounds and for protecting the wound from infection due to their healing and cell renewal properties. Very recently, they are started to be used in regeneration of nerve cells which are extremely hard to heal and it is reported that they can successfully repair the nerve cells in rats and sheep which have been broken at a level where the organism alone cannot repair.

### Technical Problems of which the Invention Aims to Solve

The main object of this invention is to prepare an extract with a high selective anti-cancer property from the spider web that has a low side effect on healthy cells and that is especially non-toxic, that does not require use of antineoplastic cancer chemotherapy agents which have genotoxic side effects on non-targeted healthy cells since it does not have selective properties currently and that can be easily obtained as the main ingredient. Another object of the invention is to provide a method for obtaining spider web extract comprising the steps of grinding the spider web by balls with liquid nitrogen in order bring it into powder form, precipitating spider web extract in a solvent containing ethyl acetate, hydrochloric acid (HCL) and dimethyl sulphoxide and thus obtaining a mixture containing a liquid extract part and a solid part; removal of solvent from the liquid extract for separating the liquid extract part from the solid part and to obtain a solid or semi-solid extract and optionally mixing the solid or semi-solid extract with a polar re-organizing solvent containing dimethyl sulphoxide, water, n-butanol, isopropanol, n-propanol, ethanol and methanol or a mixture thereof. Another object of the invention is to propose a new drug candidate in fighting the cancer and to exhibit a selective and targeted anti-cancer effect wherein said obtained spider web extract creates a DNA damage and protein damage in the prostate cancer cells by increasing the reactive oxygen species without creating a death effect on the healthy cells and then creates a programmed cell death through intrinsic and extrinsic pathways by activating the cell control pathways.

### Description of the Figures

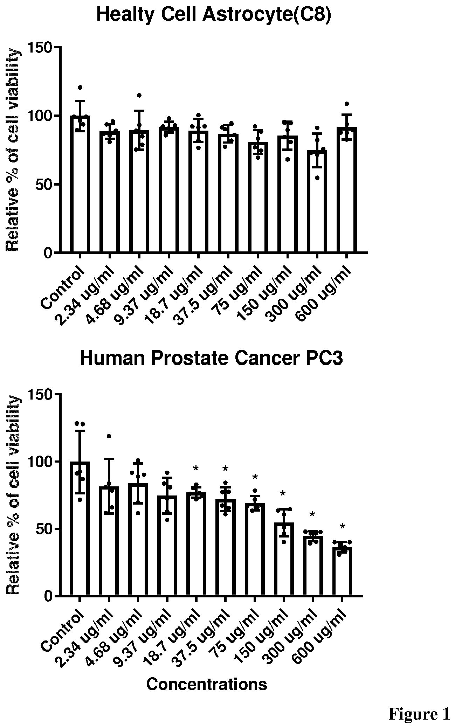

- **Figure 1.**: Graphic showing cell viability in healthy astrocyte (C8) and prostate cancer (PC3) cells treated with 9 different doses of Spider web extract obtained by serial dilution starting from the dose of 600 µg/ml for 24 hours (MTT test). * Statistically different when compared to control; one-way ANOVA, Duncan test, p ≤ 0.05.

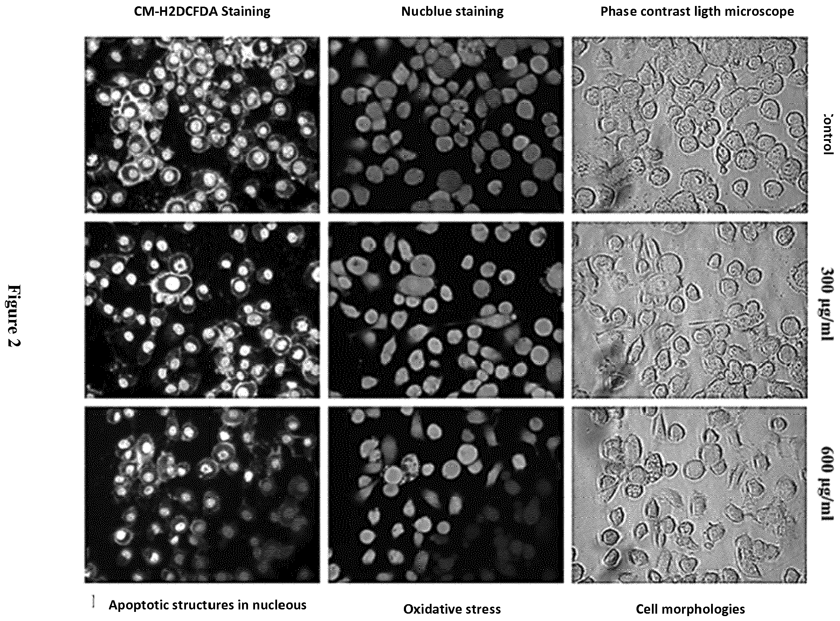

- **Figure 2.**: Microscopic view obtained by 400 x magnification showing intracellular apoptotic structures, oxidative stress and apoptosis morphology in vehicle material (Control), and in prostate cancer PC3 cells treated with spider web extract at 600 and 300 µg/ml and for 24 hours.

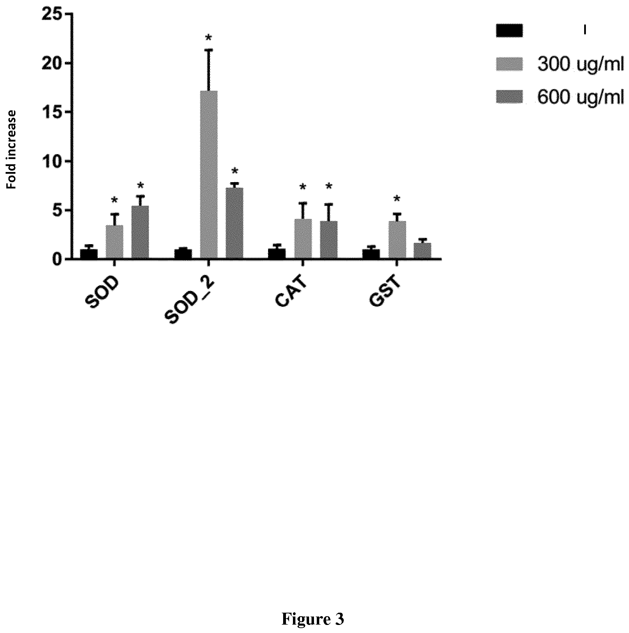

- **Figure 3.**: Graphic showing CuZn-Superoxide dismutase (SOD), Mn-Superoxide dismutase (SOD2), Catalase (CAT) and Glutathione-S-transferase (GST) gene expression in vehicle material (Control), and in prostate cancer PC3 cells subjected to spider web extract at 600 and 300 µg/ml for 24 hours. * Statistically different when compared to control; one-way ANOVA, Duncan test, p≤0.05.

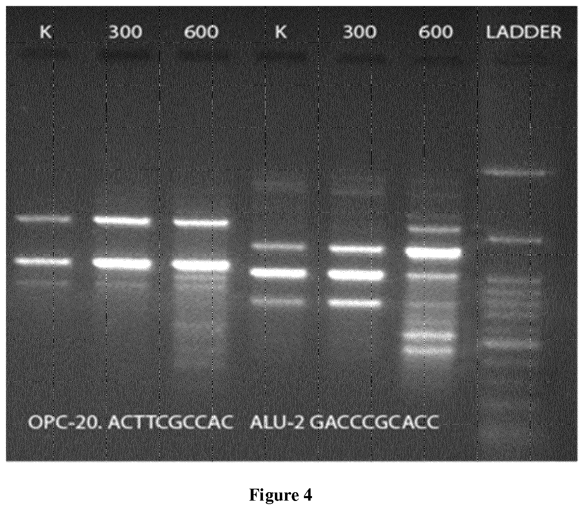

- **Figure 4.**: Result of RAPD analysis in vehicle material (Control), and in prostate cancer PC3 cells subjected to spider web extract at 600 and 300 µg/ml for 24 hours.

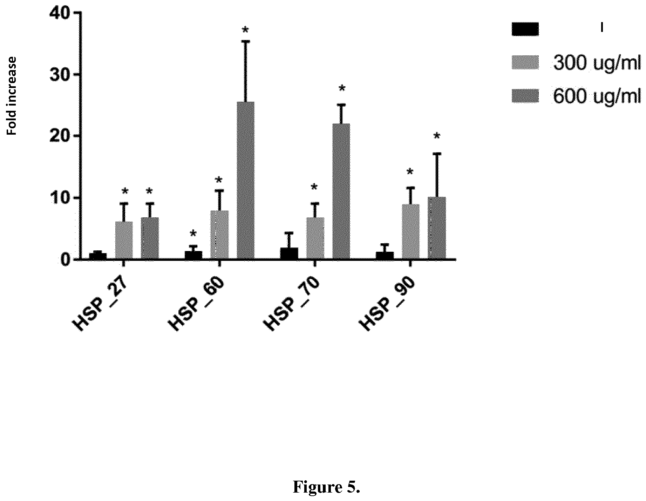

- **Figure 5**.: Graphic showing expressions related to heat shock family genes (HSP27, HSP60, HSP70 and HSP90) in vehicle material (Control), and in prostate cancer PC3 cells subjected to spider web extract at 600 and 300 µg/ml for 24 hours. * Statistically different when compared to control; one-way ANOVA, Duncan test, p≤0.05.

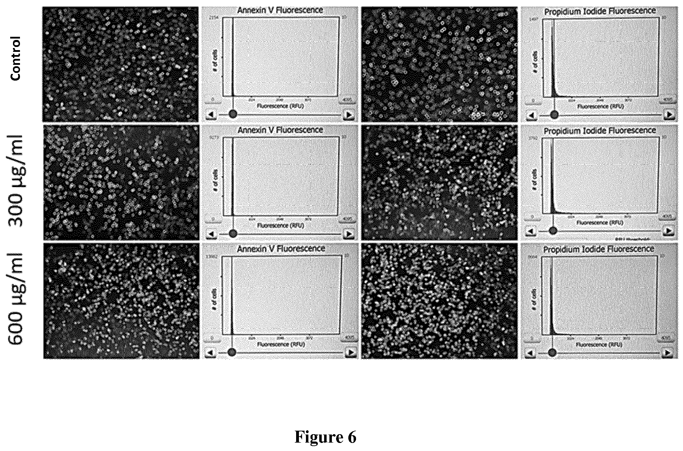

- **Figure 6.**: Cytometer view illustrating device analysis results that show apoptotic cells and RFU values obtained by TALI image-based cytometer after staining with Annexin V/Propidium Iodide in vehicle material (Control), and in prostate cancer PC3 cells subjected to spider web extract at 600 and 300 µg/ml for 24 hours.

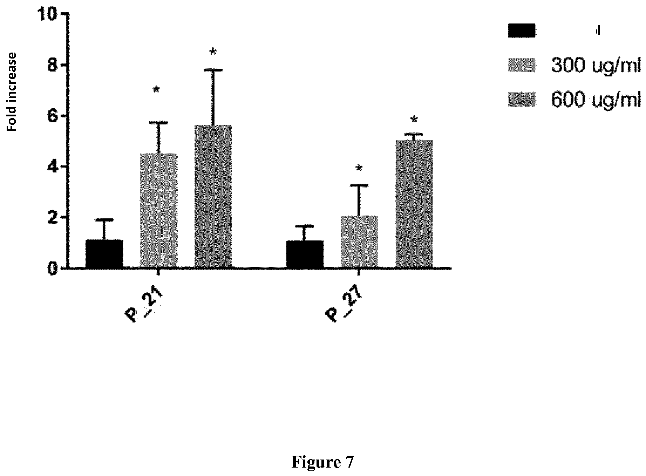

- **Figure 7.**: Graphic showing expressions of Cell cycle control point genes [Cycline dependent kinase inhibitor 1 (p21Chip1), Cycline dependent kinase inhibitor 1B (*p*27^{Kip1})] in vehicle material (Control), and in prostate cancer PC3 cells subjected to spider web extract at 600 and 300 µg/ml for 24 hours. *Statistically different when compared to control; one-way ANOVA, Duncan test, p≤0.05.

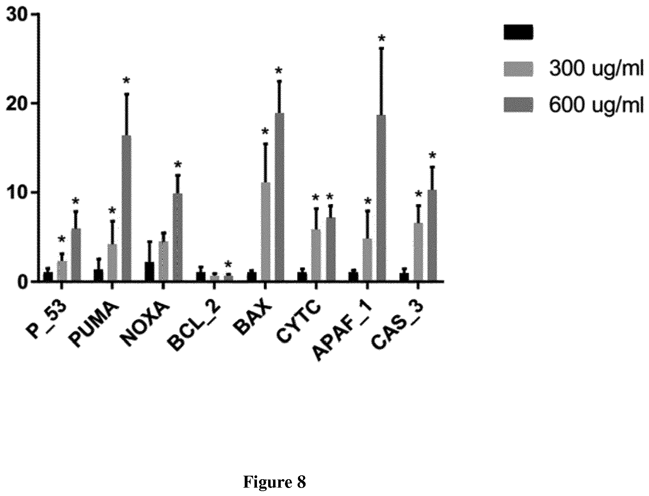

- **Figure 8.**: Graphic showing expressions of mitochondrial apoptosis pathway genes [tumor suppressor (P53), B-cell lymphoma 2 (BCL 2), BCL2 associated X (BAX), p53 upregulated modulator (PUMA), Phorbol-12-myristate-13-acetate-induced protein 1 (NOXA), cytochrome complex (Cyt-C), Apoptosis activator factor 1 (APAF1) and Caspase 3] in vehicle material (Control), and in prostate cancer PC3 cells subjected to spider web extract at 600 and 300 µg/ml for 24 hours. *Statistically different when compared to control; one-way ANOVA, Duncan test, p≤0.05.

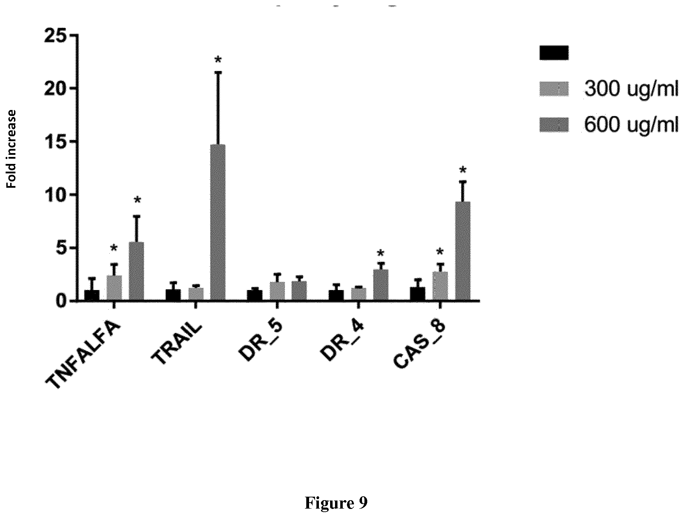

- **Figure 9.**: Graphic showing expressions of death receptor (extrinsic apoptosis) pathway genes [tumor necrosis factor alpha (TNF-α), apoptosis inducing ligand (TRAIL), death receptors (DR4 and DR5)] in vehicle material (Control), and in prostate cancer PC3 cells subjected to spider web extract at 600 and 300 µg/ml for 24 hours. *Statistically different when compared to control; one-way ANOVA, Duncan test, p≤0.05.

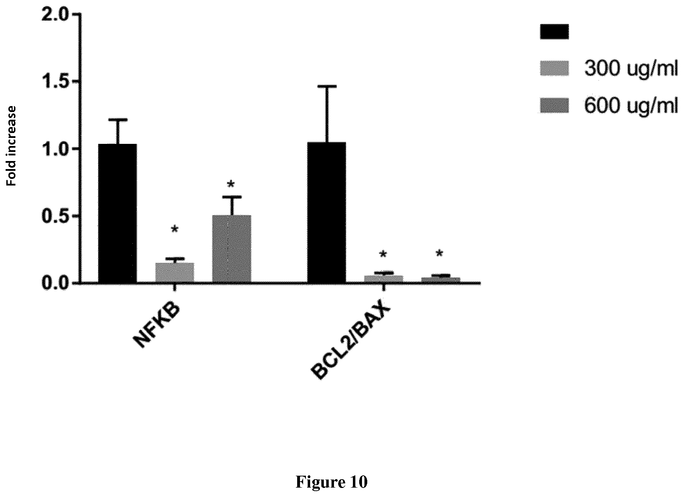

- **Figure 10**.: Graphic showing BCL2/BAX ratio that plays a role in degradation of mitochondrial membrane potential and nuclear factor kappa (NF-kB) in vehicle material (Control), and in prostate cancer PC3 cells subjected to spider web extract at 600 and 300 µg/ml for 24 hours. *Statistically different when compared to control; one-way ANOVA, Duncan test, p≤0.05.

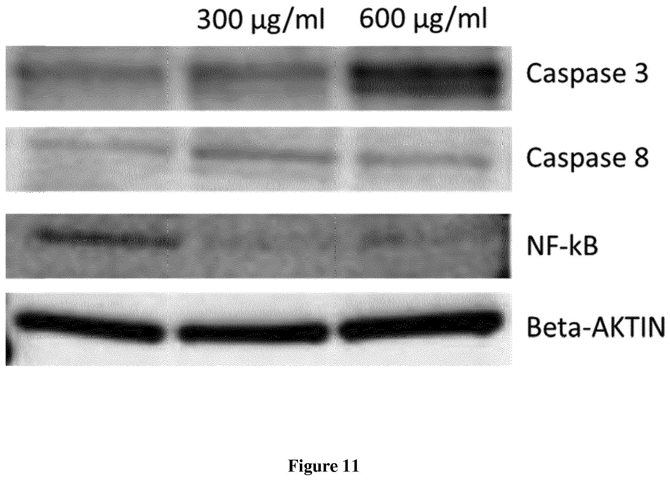

- **Figure 11.**: Chemiluminescence image showing results of western blot analysis and amount of Caspase 3, Caspase 8 and NF-kB protein in vehicle material (Control), and in prostate cancer PC3 cells subjected to spider web extract at 600 and 300 µg/ml for 24 hours.

### Description of the Invention

### Method of obtaining spider web extract

Spider web is collected directly over blackberry bushes (Rubus spp. Rosaceae)into sterile 50 cc polypropylene falcon tubes using sterile gloves. The spider webs brought to the laboratory are cleared from all plant and insect residues in a sterile chamber. The obtained web is washed with ultra-pure water for 4 times and dried under sterile environment in room conditions. The dried spider web is weighed, put into 2 ml sterile special polypropylene tubes and 2 pieces of 1 mm tungsten carbide ball and approximately 100 pieces of micro glass ball (0.01 mm diameter) are put into the tube. These tubes are immersed in liquid nitrogen and allowed to stand for 1 minute. Then, it is quickly placed into the tissue lyser (Tissue lyser LT, Qiagen) and ruptured for 15-60 minutes at the highest rpm. Rupturing process continues for an additional 45-180 minutes by respectively adding ethyl acetate, dimethyl sulfoxide and hydrochloric acid to the ground spider web. Thus, a mixture containing a liquid extract part and a solid part is obtained. Then, this mixture is allowed to stand in a heater and cooler shaker between 55-65 °C at approximately 1200 rpm for 1 hour. After this process, separation of liquid extract part from the solid part and removal of solvent from liquid extract part in order to obtain a solid or semi-solid extract are completed. Then, the obtained mixture is centrifuged for 10 minutes at approximately 1500 rpm in room temperature, the supernatant in the tubes is collected to a sterile tube. This supernatant is completely evaporated under nitrogen in room conditions and the spider web extract is obtained by mixing the resulting lyophilized material with a polar rearrangement solvent containing ultra-pure dimethyl sulphoxide, water, n-butanol, isopropanol, n-propanol, ethanol and methanol or a mixture thereof optionally in solid or semi-solid form. The extract is filtered in 0.22 µm mesh filters. For short term storage, it is stored in 1.5 ml Eppendorf tubes at -20 °C and for long term storage in cryovials at -80 °C.
The obtained spider web must contain at least 5 and in a proper extraction all of the active ingredients given in the table below within a tolerance of ±20% deviation from the values stated in the table.

| **Polyphenol Content of Spider Web Extract** | | | |
|---|---|---|---|
| **Phenolic Material** | **Retention Time Count** | | **Concentration (ng/ml)** |
| Gallic Acid | 0,534 | 1345 | 41,3478 |
| Catechin | 0,539 | 171 | 10,5757 |
| Protocatechuic acid | 0,556 | 48444 | 790,1062 |
| 2,5-Dihydroxybenzoic Acid | 0,564 | 13569 | 606,5906 |
| Trans Caffeic Acid | 0,607 | 9409 | 89,1363 |
| Trans-p-coumaric acid | 0,607 | 292 | 103,6793 |
| Naringin | 3,983 | 407 | 41,4550 |
| Quercetin | 4,130 | 27798 | 361,0320 |
| Trans Sinapic Acid | 4,138 | 38 | 195,4869 |

| **Catecholamine and Hormones** | **Retention Time Count** | | **Concentration (ng/ml)** |
|---|---|---|---|
| Melatonin | 4,186 | 81 | 3,9153 |
| Salicylic acid | 3,745 | 2109 | 42,2579 |
| Absisic Acid | 4,197 | 75 | 2,4156 |
| Jasmonic Acid | 4,373 | 14 | 1,5531 |
| Indol-3-Acetic Acid | 3,805 | 17 | 23,7142 |

### The spider web extract of the invention has the anti-cancer properties given below:

The spider web extract has a strong lethal effect on prostate cancer cells without creating a statistically significant death effect on healthy cells. This effect is selective and occurs by selecting cancer cells. The lethal effect of the spider web on prostate cancer can be 70% or higher in high doses where it is not toxic to healthy cells (Figure 1).

The spider web extract shows its anti-cancer effect on the prostate cancer cells by regulating the vital cellular death and life mechanisms such that inducing a programmed death in the cancer cells. In order;
The spider web extract, after administering to prostate cancer cells for 24 hours, causes a rapid increase in reactive oxygen species and thus creates a strong oxidative effect in the cancer cells (Figure 2), and simultaneously causes a rapid increase in antioxidant gene expressions which are the biomarker of oxidative stress (Figure 3); The spider web extract creates a substantial level of DNA damage in prostate cancer cells (Figure 4); The spider web extract causes faulty coding and misfolding of small and large protein molecules in prostate cancer cells and also causes an increase in HSPs (heat shock) gene expression which is the biomarker of protein-dependent stress (Figure 5); then, it stops G1/S phase transition by triggering *p*21^{Cip1} and *p*27 ^{Kip1}genes which are cell cycle control pathway genes (Figure 7), at the same time it activates the P53 tumor suppressor gene by suppressing NF-kB gene and protein expression (Figure 10, Figure 11, respectively), this activation inhibits PUMA and NOXA pathways and BCL-2 genes, simultaneously it activates the BAX gene (Figure 8), and the rapidly decreasing BCL-2/BAX ratio (Figure 10) degrades the membrane structure of the mitochondria, Cyt-C flows into cytosole and it creates the apoptosome complex here by combining with APAF1, then the spider web extract leads the prostate cancer cells to a programmed death through an intrinsic apoptosis pathway (Mitochondrial apoptosis pathway) as a result of rapidly increasing Caspase 3 gene (Figure 8) and protein expression (Figure 11). Similarly, by the TRAIL gene effect, the spider web extract activates especially DR4 and DR5, triggers Caspase 8 gene expression (Figure 9), and thus leads to a programmed cell death in prostate cancer cells by extrinsic apoptosis pathway (death receptor signaling) activation through Caspase 3 pathway (Figure 6).

The spider web extract of the invention and its anti-cancer effect cannot be limited to what is described herein. It is apparent to those who are skilled in the art that the amount and ratio of additives, their preparation methods, the method of mixing the mixture and the dose and times of used spider web extract may vary. It is apparent that a person skilled in the art can reproduce the novelty presented in the invention by using similar embodiments and/or can apply this embodiment to similar fields used in the related technique. Thus, it will be apparent that such embodiments shall lack the criteria of novelty and especially exceeding the known state of the art.

### The Method of Application of the Invention to Industry

Today, the effort for developing cancer drugs and also the market of using natural products are financed with a huge budget expressed by billion dollars on the global scale. Scientific and technological developments, Nobel prizes awarded in the healthcare field in the last two years, approximately 75% of all the currently approved anti-cancer chemotherapy agents being of the natural product origin increase the importance and market value of the natural products in the fight with cancer.

At least in accordance with our literature research data, the anti-cancer effect of the spider web extract on the prostate cancer cells is proposed for the first time by this patent application on the world. Besides being a natural product, today the spider web extract production is successfully established and executed for providing raw material to industrial scale productions in the production farms located in the United States of America and Australia. Thus, as in several natural products, dependence to nature can be substantially reduced for also this natural product. This is an additional value for our product.

Besides, as our invention can be used as an anti-cancer agent alone, it also presents highly important target molecules in development of developing a successful chemotherapy drug with its selective anti-cancer effect, all of the genes in the life and death pathways it affects or regulates on programmed cell death in the cell or with its separate effects. Moreover, the specific cell pathways it affects enable development of combined application with a plurality of chemotherapy drugs in order to increase the effects of other chemotherapy agents.

## Claims

1. A method of obtaining spider web extract, comprising the steps of;
• collecting the spider web over the shrubs and bushes that do not contact ground, preferably over Rubus spp. (Rosaceae) by sterile equipment, cleaning and washing with pure water and drying in the laboratory, freezing in liquid nitrogen and grinding in a tissue lyser with balls,
• continuing the lysis process by adding at least one of hydrochloric acid, dimethyl sulphoxide, ethyl acetate chemicals;
• thus, obtaining a mixture containing a liquid extract fraction and a solid fraction,
• separating the liquid extract fraction from the solid fraction by mixing in the heater and cooler shaker and then allowing to rest at 55-65 °C for 1-4 hours and extracting the solvent from the liquid extract fraction to obtain a solid or semi-solid extract,
• centrifuging the obtained mixture at room temperature, collecting the upper phase liquid fraction, evaporating said fraction and then mixing the obtained lyophilized material with a polar rearrangement solvent containing dimethyl sulphoxide, water, n-butanol, isopropanol, n-propanol, ethanol and methanol or a mixture thereof, optionally to a solid or semi-solid state.

2. Spider web extract related to the method described in Claim 1, **characterized by** killing prostate cancer cells by creating oxidative stress.

3. Spider web extract related to the method described in Claim 1 that is used as selective Nuclear Factor kappa B (NF-kB) inhibitor in prostate cancer cells.

4. Spider web extract related to the method described in Claim 1, **characterized by** acting selectively on prostate cancer cells as tumor suppressor (P53), p53 upregulated modulator (PUMA), Phorbol-12-myristate-13-acetate-induced protein 1 (NOXA), B-cell lymphoma 2 (BCL 2) associated X (BAX), Apoptosis activator factor 1 (APAF1) and Caspase 3 activator and B-cell lymphoma 2 (BCL-2) inhibitor, respectively and thus being used as mitochondrial programmed cell death activator.

5. Spider web extract related to the method described in Claim 1, **characterized by** being used as programmed cell death activator on prostate cancer cells through extrinsic (death receptors) apoptosis pathway by activation of apoptosis inducing ligand associated to TNF (TRAIL), death receptor 4 (DR4), death receptor 5 (DR5) and Caspase 8, respectively.

6. Spider web extract related to the method described in Claim 1, which can be used in treatment and prevention of prostate cancer.
